# EUROPEAN PATENT APPLICATION

(11) **EP 4 393 530 A1**
(43) Date of publication of application: **03.07.2024**
(21) Application number: 23218305.3
(22) Date of filing: 19.12.2023
(51) Int. Cl.: A61M 5/315, A61B 5/15

(54) **SYRINGE DEVICE PLUNGER WITH AXIALLY EXTENDABLE DETENTS AND METHODS OF USING AND PROCESSES OF MANUFACTURING THE SAME**

(30) Priority: 26.12.2022 US 202263477209 P
(71) Applicant: Estar Technologies Ltd, 5885111 Holon (IL)
(72) Inventor: ESTERON, Aaron, 5839106 Holon (IL)
(74) Representative: Pearl Cohen Zedek Latzer Baratz UK LLP

(57) **Abstract**

A syringe device with plunger having axially biased detents including: a syringe tube, a plunger including: an essentially elongated shaft; a piston, a thumb rest, at least one recess in the essentially elongated shaft of the plunger, in which the recess includes an interior void volume, oriented essentially orthogonally to a longitudinal centerline of the essentially elongated shaft of the plunger, at least one detent assembly including: a stopper element, accommodatable within the at least one recess in the essentially elongated shaft of the plunger; a biasing means, configured for spontaneously driving the stopper element outwardly, and at least one retaining element, configured for restricting a movement of the stopper element within the at least one recess; in which the stopper element is axially translatable within the at least one recess, in a direction essentially orthogonal to the longitudinal centerline of the essentially elongated shaft of the plunger.

## Description

### TECHNICAL FIELD

In general, the present invention pertains to the art of medical devices. In particular, the invention relates to a syringe device with plunger characterized by axially extendable detents and methods of using and manufacturing the same.

### BACKGROUND ART

It is believed that the current state of the art is represented by the following patent literature: US5215536, US9561327 and WO9101768.

US5215536 that is believed to represent the closest prior art discloses a syringe for injecting medicinal fluids into a patient or aspirating fluids or tissue from a patient, wherein the syringe incorporates a self-locking mechanism on the plunger thus preventing the plunger from being withdrawn back into the barrel by the action of a vacuum in the syringe barrel. In US5215536, the self-locking mechanism is independent of the relative rotational relationship of the plunger with respect to the barrel. In US5215536, unlocking may be effected by simply squeezing the self-locking mechanism on the plunger, no rotational force being necessary.

### SUMMARY OF THE INVENTION

The following summary of the invention is provided to exhibit the basic understanding of some principles, underlying various aspects and features of the invention. This summary is not an extensive overview of the invention and as such it is not necessarily intended to particularly identify all key or critical elements of the invention and is not to delineate the scope of the invention. Its sole purpose is to present some concepts of the invention in a simplified form as a prelude to the following more detailed.

The invention was made in view of the deficiencies of the prior art and provides systems, methods and processes for overcoming these deficiencies. In accordance with some embodiments and aspects of the present invention a syringe device with plunger having axially biased detents includes: (a) a syringe tube including: (I) an essentially elongated cylindrical shell shaped barrel; (II) a frusto-conical tip, at a distal portion of the barrel; (III) at least one finger flange, at a proximal portion of the barrel, configured for manual grip; (b) a plunger including: (I) an essentially elongated shaft; (II) a piston at a distal portion of the elongated shaft, including at least one sealing element, configured to form a sealing arrangement with an interior surface of the barrel, whilst contiguously translatable therein; (III)a thumb rest at a distal portion of the elongated shaft, configured for exerting a manual pressing force, onto the plunger; (IV) at least one recess in the essentially elongated shaft of the plunger, in which the recess includes an interior void volume, oriented essentially orthogonally to a longitudinal centerline of the essentially elongated shaft of the plunger; (c) at least one detent assembly including: (I) a stopper element, accommodatable within the at least one recess in the essentially elongated shaft of the plunger; (II) a biasing means, configured for spontaneously driving the stopper element outwardly from the at least one recess in the essentially elongated shaft of the plunger; (III) at least one retaining element, configured for restricting a movement of the stopper element within the at least one recess in the essentially elongated shaft of the plunger, thereby preventing the stopper element from leaving the at least one recess; in which the stopper element of the at least one detent assembly is axially translatable within the at least one recess, in a direction essentially orthogonal to the longitudinal centerline of the essentially elongated shaft of the plunger.

In some embodiments, the piston of the plunger includes a fastener part, whereas the distal portion of the elongated shaft includes a respective fastener part, whereby the piston of the plunger is controllably connectable to and disconnectable from the distal portion of the elongated shaft of the plunger. In some embodiments, the fastener includes a screw threading.

In some embodiments, the at least one detent assembly includes a first detent assembly and a second detent assembly, in which the first detent assembly is disposed towards the distal portion of the shaft, whereas the second detent assembly is disposed towards the proximal portion of the shaft.

In some embodiments, the stopper element, of the at least one detent assembly, includes a structured top surface.

In some embodiments, the structured top surface of the stopper element includes an elevation at a distal portion thereof.

In some embodiments, the retaining element is selected from the group consisting of: a pawl, notch, recess, groove and snap.

In some embodiments, the retaining element is selected from the group consisting of: a retaining element forming an integral part of the essentially elongated shaft of the plunger and a retaining element forming an integral part of the stopper element.

In some embodiments, the retaining element further includes an external retaining element, which forms an integral part neither of the essentially elongated shaft of the plunger nor of the stopper element.

In accordance with some embodiments and aspects of the present invention, a process of manufacturing a syringe device with plunger having axially biased detents includes the steps of: (a) pre-manufacturing a syringe tube including: (I) an essentially elongated cylindrical shell shaped barrel; (II) a frusto-conical tip, at a distal portion of the barrel; (III) at least one finger flange, at a proximal portion of the barrel, configured for manual grip; (b) molding a plunger including: (I) providing a plunger mold embodying a shape of: (i) an essentially elongated shaft; (ii) a distal portion of the elongated shaft, including at least one element for anchoring a sealing element; (iii) a thumb rest at a distal portion of the elongated shaft, configured for exerting a manual pressing force, onto the plunger; (iv) at least one recess in the essentially elongated shaft of the plunger, in which the recess includes an interior void volume, oriented essentially orthogonally to a longitudinal centerline of the essentially elongated shaft of the plunger; (v) at least one structural element, configured for restricting a movement of a stopper element within the at least one recess; (II) injecting a melted polymeric resin into the plunger mold; (c) pre-manufacturing a piston, including at least one sealing element, configured to form a sealing arrangement with an interior surface of the barrel, whilst contiguously translatable therein; (d) mounting the piston onto the distal portion of the elongated shaft of the plunger; (e) molding a stopper element including: (I) providing a stopper mold embodying a shape of: (i) at least one stopper element accommodatable within the at least one recess in the essentially elongated shaft of the plunger; (ii) at least one structural retaining element, configured for restricting the movement of the stopper element within the at least one recess in the essentially elongated shaft of the plunger; (II) injecting a melted polymeric resin into the stopper mold; (f) pre-manufacturing a biasing means, configured for spontaneously driving the stopper element outwardly from the at least one recess in the essentially elongated shaft of the plunger; (g) assembling at least one detent assembly including: (I) inserting the biasing means into the at least one recess in the essentially elongated shaft of the plunger; (II) inserting the stopper element into the at least one recess in the essentially elongated shaft of the plunger; (III) securing the stopper element within the at least one recess in the essentially elongated shaft of the plunger, by forming an operational connection between the at least one structural retaining element the at least one recess in the essentially elongated shaft of the plunger and the at least one structural retaining element of the stopper element, thereby restricting a movement of the stopper element within the at least one recess and thereby preventing the stopper element from leaving the at least one recess in the essentially elongated shaft of the plunger; whereby the stopper element of the at least one detent assembly is axially translatable within the at least one recess, in a direction essentially orthogonal to the longitudinal centerline of the essentially elongated shaft of the plunger.

In accordance with some embodiments and aspects of the present invention, a method of harvesting biological material includes the steps of: (a) providing a syringe device including: (I) a syringe tube including: (i) an essentially elongated cylindrical shell shaped barrel; (ii) a frusto-conical tip, at a distal portion of the barrel, and (iii) at least one finger flange, at a proximal portion of the barrel, configured for manual grip; (II) a plunger including: (i) an essentially elongated shaft; (ii) a piston at a distal portion of the elongated shaft, including at least one sealing element, configured to form a sealing arrangement with an interior surface of the barrel, whilst contiguously translatable therein; (iii) a thumb rest at a distal portion of the elongated shaft, configured for exerting a manual pressing force, onto the plunger, and (iv) at least one recess in the essentially elongated shaft of the plunger, in which the recess includes an interior void volume, oriented essentially orthogonally to a longitudinal centerline of the essentially elongated shaft of the plunger; (III) at least one detent assembly including: (i) a stopper element, accommodatable within the at least one recess in the essentially elongated shaft of the plunger; (ii) a biasing means, configured for spontaneously driving the stopper element outwardly from the at least one recess in the essentially elongated shaft of the plunger, and (iii) at least one retaining element, configured for restricting a movement of the stopper element within the at least one recess in the essentially elongated shaft of the plunger, thereby preventing the stopper element from leaving the at least one recess; (b) inserting a tip of a harvesting utensil into a target biological tissue; (c) connecting the frusto-conical tip of the syringe tube, to the harvesting utensil; (d) creating a negative pressure within the syringe tube, by drawing the plunger outwardly from the syringe tube, whilst the tip of the harvesting utensil is disposed within the target biological tissue; (e) drawing the plunger outwardly from the syringe tube; (f) axially translating the stopper element by the biasing means, outwardly from the at least one recess in the essentially elongated shaft of the plunger from, in the direction essentially orthogonal to the longitudinal centerline of the essentially elongated shaft of the plunger, (g) restricting by the retaining element the movement of the stopper element outwardly from the at least one recess in the essentially elongated shaft of the plunger; (h) releasing the plunger, subsequently to the axially translating the stopper element, thereby sustaining the negative pressure within the syringe tube; (i) manipulating the tip of the harvesting utensil within the target biological tissue, whilst sustaining the negative pressure within the syringe tube, thereby harvesting the biological material.

### DEFINITIONS

The term matching or a term similar thereto, as referred to herein, is to be construed as having a cross-sectional area and/or shape of a component equal or essentially similar to a cross-sectional area and/or shape of another component. It should be acknowledged that the components may only to be similar in the cross-sectional areas and/or shapes, to satisfy the term matching or similar, so long as the cross-sectional areas of the components can be mated and/or inserted into each other and/or the combination thereof essentially fits together and/or occupy essentially the same space.

The term structured, as referred to herein, is to be construed as including any geometrical shape, exceeding in complexity a plain linear shape or a shape embodying a simple and/or standardized circular, elliptical or polygonal contour or profile. Any more complex shape than a plain linear shape or a shape embodying a simple and/or standardized circular, elliptical or polygonal contour or profile, constitutes an example of structured geometry.

The term modular, as referred to herein, should be construed as a including a stand-alone and/or autonomically functioning of structured unit. The term modular *inter alia* means a standardized unit that may be conveniently installed or deployed without significant impact to the environment. The term modular, however, doesn't necessarily mean providing for ease of interchange or replacement. The term modular is optionally satisfied solely by providing for ease of onetime deployment or installation.

The term readily connectable, as referred to herein, should be construed as including any structure and/or member that is configured to be conveniently connected to other structure and/or member and/or components of a larger system or assembly. The term readily connectable, however, doesn't necessarily mean readily disconnectable or removable. The term readily connectable is optionally satisfied by providing for ease of onetime connection or coupling.

The term biasing means or alike, as referred to herein, should be construed as including any material, structure or mechanism, configured to accumulate mechanical energy, by changing the configuration thereof, upon a force exerted thereon, such as a compressive, tensile, shear or torsional force, as well as for releasing the energy accumulated therein, by returning to the normal or default configuration thereof and thereby performing a mechanical work, typically by linear or radial displacement. Examples of biasing means in a non-limiting manner include, springs, elastomers, leaf-springs, coil-springs, tension/extension spring, compression spring torsion spring, constant spring, variable spring, variable stiffness spring, flat spring, machined spring, serpentine spring, garter spring, cantilever spring, helical spring, hollow tubing springs, volute spring, V-spring, belleville washer or belleville spring, constant-force spring, gas spring, mainspring, negator spring, progressive rate coil springs, rubber band, spring washer and wave spring.

By operationally connected and operably coupled or similar terms used herein is meant connected in a specific way (e.g., in a manner allowing fluid to move and/or electric power or signal to be transmitted) that allows the disclosed system and its various components to operate effectively in the manner described herein.

The term fluid or liquid, as referred to herein, is to be construed as any material that deforms when a shear stress is applied. While fluid generally would refer to any liquids or gases, it may be used herein to describe fluidized solids and bulk solids and/or granulate matter that are capable of flowing or otherwise moving inside a device as a result of pressure differences and/or gravitational force. Such materials may include slurries, suspensions, pastes, powders, granular solids, particle solids, granulate matter, particulate matter, as well as any combinations thereof.

The term dry powder, as referred to herein, may include any substance comprising one or a plurality of constituents or ingredients with one or a plurality of (average) particulate size ranges. The term low-density dry powder means dry powders having a density of about 0.8 gram per cubic centimeter or less. In particular embodiments, the low-density powder may have a density of about 0.5 gram per cubic centimeter or less. The dry powder may be with or without cohesive or agglomeration tendencies.

The terms firm rigid, or stiff, as referred to herein, are to be construed as having rigidity modulus value, otherwise referred to as the shear modulus, of 4800 MPa or more. Materials are considered to be firm rigid, or stiff but not tensile, when such materials are incapable of being efficiently elastically flexed or bent. Stiff materials, such as steel, are defined as having rigidity modulus value well exceeding 4800 MPa.

The terms pliable or pliant, as referred to herein, are to be construed as having high tensile strength and capable of being efficiently elastically flexed or bent but not being resilient and incapable of being efficiently stretched or expanded. The term tensile or tensile strength, as referred to herein, is to be construed inter alia as a shortcut of the known term ultimate tensile strength, frequently represented acronym as UTS, meaning an intensive property of a material or structure to withstand loads tending to elongate, namely to resist tension, defined as the maximum stress that a material can withstand while been stretched or pulled before sustaining breaking, substantial deformation and/or necking before fracture, such as nylon, relating to essentially non-ductile materials, having UTS value ranging between about 600 and 1000 MPa or more, but not including rigid, firm or stiff materials.

The terms elastic or resilient, as referred to herein, are to be construed as having tensile strength lower than aforesaid tensile strength of pliable or pliant material and optionally being capable of efficiently stretching or expanding, relating *inter alia* to essentially ductile materials, having UTS value lesser than about 600 MPa.

The term slurry, as referred to herein, is to be construed as a mixture of solids denser than water suspended in liquid, usually water. Solids concentrations in a slurry typically range between about 0.5 percent and about 5 percent.

The terms method and process as used herein are to be construed as including any sequence of steps or constituent actions, regardless of a specific timeline for the performance thereof. The particular steps or constituent actions of any given method or process are not necessarily in the order they are presented in the claims, description or flowcharts in the drawings, unless the context clearly dictates otherwise. Any particular step or constituent action included in a given method or process may precede or follow any other particular step or constituent action in such method or process, unless the context clearly dictates otherwise. Any particular step or constituent action and/or a combination thereof in any method or process may be performed iteratively, before or after any other particular step or action in such method or process, unless the context clearly dictates otherwise. Moreover, some steps or constituent actions and/or a combination thereof may be combined, performed together, performed concomitantly and/or simultaneously and/or in parallel, unless the context clearly dictates otherwise. Moreover, some steps or constituent actions and/or a combination thereof in any given method or process may be skipped, omitted, spared and/or opted out, unless the context clearly dictates otherwise.

In the specification or claims herein, any term signifying an action or operation, such as: a verb, whether in base form or any tense, gerund or present/past participle, is not to be construed as necessarily to be actually performed but rather in a constructive manner, namely as to be performed merely optionally or potentially.

The term substantially as used herein is a broad term, and is to be given its ordinary and customary meaning to a person of ordinary skill in the art (and is not to be limited to a special or customized meaning), and refers without limitation to being largely but not necessarily entirely of that quantity or quality which is specified.

The term essentially means that the composition, method or structure may include additional ingredients, stages and or parts, but only if the additional ingredients, the stages and/or the parts do not materially alter the basic and new characteristics of the composition, method or structure claimed.

As used herein, the term essentially changes a specific meaning, meaning an interval of plus or minus ten percent (± 10%). For any embodiments disclosed herein, any disclosure of a particular value, in some alternative embodiments, is to be understood as disclosing an interval approximately or about equal to that particular value (i.e., ± 10%).

As used herein, the terms about or approximately modify a particular value, by referring to a range equal to the particular value, plus or minus twenty percent (+/-20%). For any of the embodiments disclosed herein, any disclosure of a particular value, can, in various alternate embodiments, also be understood as a disclosure of a range equal to about that particular value (i.e. +/-20%).

As used herein, the term or is an inclusive or operator, equivalent to the term and/or, unless the context clearly dictates otherwise; whereas the term and as used herein is also the alternative operator equivalent to the term and/or, unless the context clearly dictates otherwise.

It should be understood, however, that neither the briefly synopsized summary nor particular definitions hereinabove are not to limit interpretation of the invention to the specific forms and examples but rather on the contrary are to cover all modifications, equivalents and alternatives falling within the scope of the invention.

### DESCRIPTION OF THE DRAWINGS

The present invention will be understood and appreciated more comprehensively from the following detailed description taken in conjunction with the appended drawings in which:
**FIG 1** is a perspective view of a syringe device with plunger having axially biased detents, according to some embodiments of the present invention;
**FIG 2A** is an exploded view of the syringe device with plunger having axially biased detents, according to some embodiments of the present invention;
**FIG 2B** is an enlarged view of a detent assembly of the syringe device, according to some embodiments of the present invention;
**FIG 3** is a flowchart of a process of manufacturing a syringe device with plunger having axially biased detents, according to some embodiments of the present invention;
**FIG 4** is a flowchart of a method of harvesting biological material, according to some embodiments of the present invention;
**FIG 5** is a flowchart of an exemplary procedure of bone marrow harvesting with the syringe device, according to some embodiments of the present invention;
**FIG 6** is a flowchart of an exemplary procedure of bone marrow concentration with the syringe device, according to some embodiments of the present invention.

The drawings are not necessarily complete and components are not necessarily to scale.

### DETAILED DISCLOSURE OF EMBODIMENTS

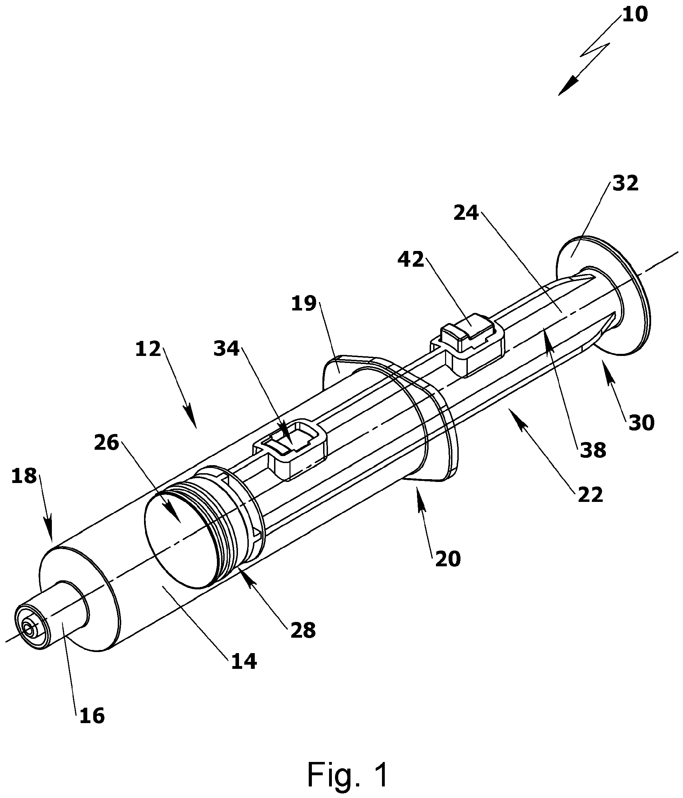

In accordance with some embodiments of the present invention, reference is now made to **FIG 1** to **2B**, showing syringe device **10** with plunger having axially biased detents. In some embodiments, syringe device **10** comprises syringe tube **12.** Syringe tube **12** comprises essentially elongated cylindrical shell shaped barrel **14.**

In some embodiments, syringe tube **12** further comprises frusto-conical tip **16** at distal portion **18** of essentially elongated cylindrical shell shaped barrel **14.** In some embodiments, syringe tube **12** further comprises at least one finger flange **19** at proximal portion **20** of essentially elongated cylindrical shell shaped barrel **14.**

In some embodiments, syringe device **10** further comprises plunger **22.** Plunger **22** comprises essentially elongated shaft **24.** In some embodiments, syringe device **10** further comprises piston **26** at distal portion **28** of elongated shaft **24.** Piston **26** comprises at least one sealing element. At least one sealing element of plunger **22** is configured for forming a sealing arrangement with an interior surface of barrel **14,** whilst contiguously translatable within barrel **14.**

In some embodiments, piston **26** of plunger **22** comprises a fastener moiety, whereas distal portion **28** of elongated shaft **24** comprises respective fastener moiety **29,** whereby piston **26** of plunger **22** is controllably connectable and disconnectable to and from distal portion **28** of elongated shaft **24.** In some examples, the fastener moiety of piston **26** of plunger **22** and/or fastener moiety **29** at distal portion **28** of elongated shaft **24** comprise/s a screw threading.

In some embodiments, plunger **22** further comprises thumb rest **32** at proximal portion **30** of elongated shaft **24.** Thumb rest **32** is configured for exerting a manual pressing force, onto plunger **22.** In some embodiments, plunger **22** further comprises at least one recess **34** in essentially elongated shaft **24** thereof. At least one recess **34** comprises an interior void volume. The interior void volume is oriented essentially orthogonally in the direction of arrow **36** to longitudinal centerline **38** of essentially elongated shaft **24** of plunger **22.**

In some embodiments, syringe device **10** further comprises at least one detent assembly **40.** At least one detent assembly **40** comprises stopper element **42.** Stopper element **42** is accommodatable within at least one recess **34** in essentially elongated shaft **24** of plunger **22.** In some embodiments, stopper element **46** of at least one detent assembly **40** is axially translatable within at least one recess **34** essentially orthogonally to longitudinal centerline **38** of essentially elongated shaft **24** of plunger **22.**

In some embodiments, stopper element **42** comprises structured top surface **43.** Structured top surface **43** of stopper element **42** exemplarily comprises elevation **45** at a distal portion thereof.

In some embodiments, at least one detent assembly **40** further comprises a plurality or pair of detent assemblies, wherein a first detent assembly is disposed towards distal portion **28** of elongated shaft **24** of plunger **22** and a second detent assembly is disposed towards proximal portion **30** of elongated shaft **24** of plunger **22.**

In some embodiments, at least one detent assembly **40** further comprises biasing means **44.** Biasing means **44** is configured for spontaneously driving stopper element **42** outwardly from at least one recess **34** in essentially elongated shaft **24** of plunger **22.** Biasing means **44** is configured for spontaneously driving stopper element **42** outwardly from at least one recess **34** in essentially elongated shaft **24** of plunger **22,** in the direction of arrow **36** essentially orthogonally to longitudinal centerline **38** of essentially elongated shaft **24** of plunger **22**

In some embodiments, at least one detent assembly **40** further comprises retaining element **46.** Retaining element **46** is configured for restricting a movement of stopper element **42** within at least one recess **34** in essentially elongated shaft **24** of plunger **22,** thereby preventing stopper element **42** from leaving at least one recess **34.** In some examples, retaining element **46** is a pawl and/or notch and/or recess and/or groove.

In some embodiments, retaining element **46** forms an integral part of essentially elongated shaft **24** of plunger **22** and of stopper element **42.** In some embodiments, retaining element **46** is a combination of elements forming an integral part of essentially elongated shaft **24** of plunger **22** and/or of stopper element **42.**

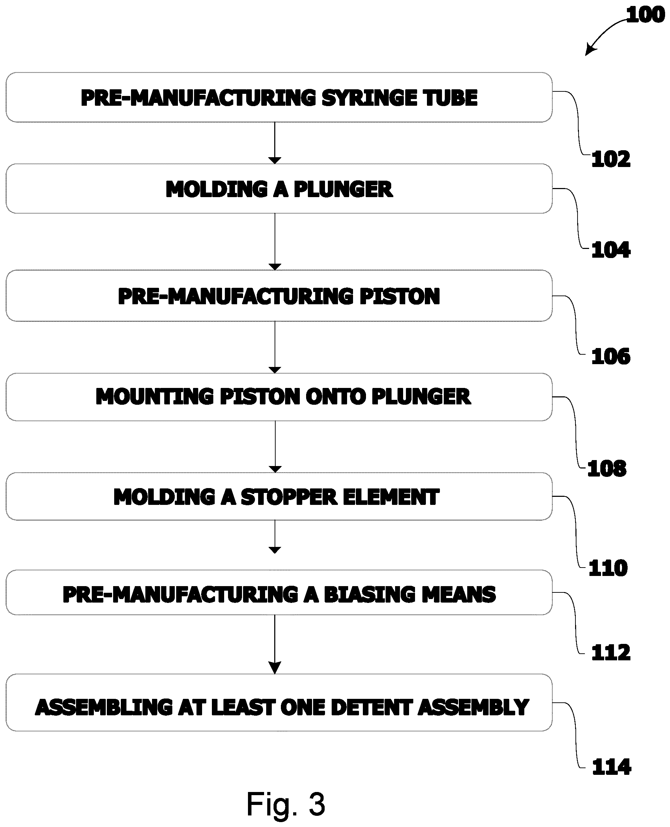

In accordance with some embodiments of the present invention, reference is now made **FIG 3** showing a flowchart of process **100** of manufacturing a syringe device with plunger having axially biased detents. The process of the embodiment of **FIG 3** illustrates various features that may be interchangeable with elements of any other embodiment described in the specification.

In some embodiments, process **100** commences at step **102** of pre-manufacturing or otherwise procuring a syringe tube, such as syringe tube **12** shown in **FIG 1****.** The syringe tube typically comprises an essentially elongated cylindrical shell shaped barrel, a frusto-conical tip and at least one finger flange, such as essentially elongated cylindrical shell shaped barrel **14,** frusto-conical tip **16** and at least one finger flange **20** shown in **FIG 1****.**

In some embodiments, process **100** further proceeds to step **104** of molding a plunger. Step **104** includes a step of providing or shaping a plunger mold. In some embodiments, the plunger mold embodies a shape of an essentially elongated shaft, with a distal portion of the elongated shaft, such as essentially elongated shaft **24** with distal portion **28** shown in **FIG 1****.** In some embodiments, the plunger mold further embodies a shape of a thumb rest at a proximal portion of the elongated shaft, such as thumb rest **32** shown in **FIG 1****.**

In some embodiments, the plunger mold further embodies a shape of at least one recess in the essentially elongated shaft of the plunger, such as at least one recess **34** shown in **FIG 1****,** and of at least one structural retaining element configured for restricting the movement of a stopper element within at least one recess. In some embodiments, step **104** of molding a plunger further includes injecting a melted polymeric resin into the plunger mold.

In some embodiments, process **100** further comprises step **106** of pre-manufacturing a piston, such as piston **26** shown in **FIG 1****.** The piston comprises at least one sealing element, configured to form a sealing arrangement with an interior surface of the barrel, whilst contiguously translatable therein. In some embodiments, process **100** further includes step **108** of mounting the piston onto the distal portion of the elongated shaft of the plunger.

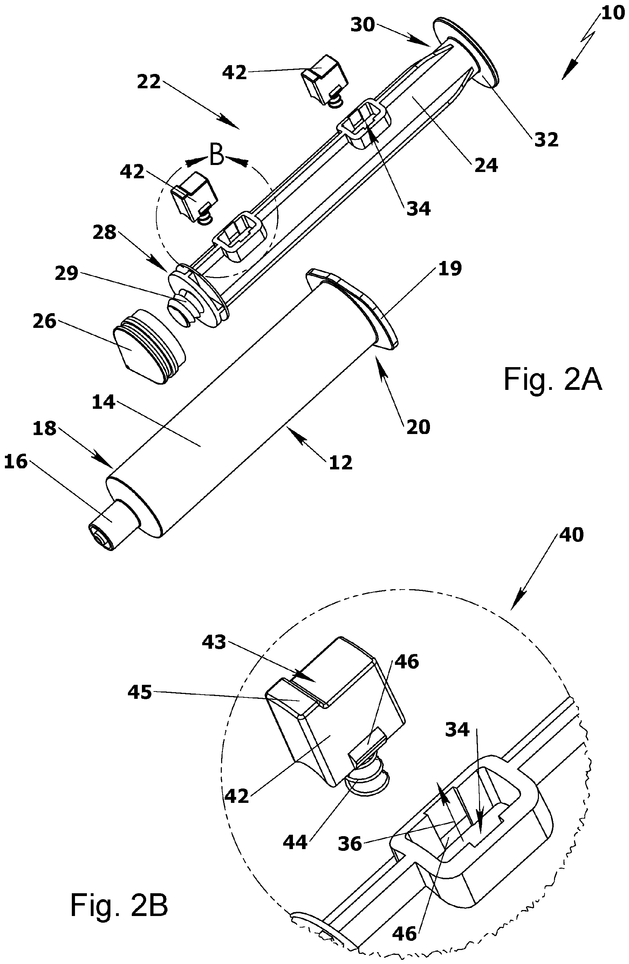

In some embodiments, process **100** further includes step **110** of molding a stopper element, such as stopper element **42** shown in **FIG 1****.** In some embodiments, step **110** comprises providing a stopper mold embodying a shape of at least one stopper element accommodatable within at least one recess in the essentially elongated shaft of the plunger. The stopper element further embodies a shape of at least one structural retaining element, such as retaining element **46** shown in **FIG 2B****,** configured for restricting the movement of the stopper element within at least one recess in the essentially elongated shaft of the plunger. In some embodiments, step **110** further includes injecting a melted resin into the stopper mold.

In some embodiments, process **100** further comprises step **112** of pre-manufacturing a biasing means, such as biasing means **44** shown in **FIG 2B****.** The biasing means configured for spontaneously driving the stopper element outwardly from at least one recess in the essentially elongated shaft of the plunger. In some embodiments, the stopper element of at least one detent assembly is axially translatable within at least one recess, in a direction essentially orthogonal to the longitudinal centerline of the essentially elongated shaft of the plunger.

In some embodiments, process **100** further includes step **114** of assembling at least one detent assembly, such as detent assembly **40** shown in **FIG 2B****.** In some embodiments, step **114** includes inserting the biasing means into at least one recess in the essentially elongated shaft of the plunger and inserting the stopper element into at least one recess in the essentially elongated shaft of the plunger.

In some embodiments, step **114** further includes securing the stopper element within at least one recess in the essentially elongated shaft of the plunger, by forming an operational connection between at least one structural retaining element of the recess in the essentially elongated shaft of the plunger and at least one structural retaining element of the stopper element, thereby restricting a movement of the stopper element within at least one recess and thereby preventing the stopper element from leaving at least one recess in the essentially elongated shaft of the plunger.

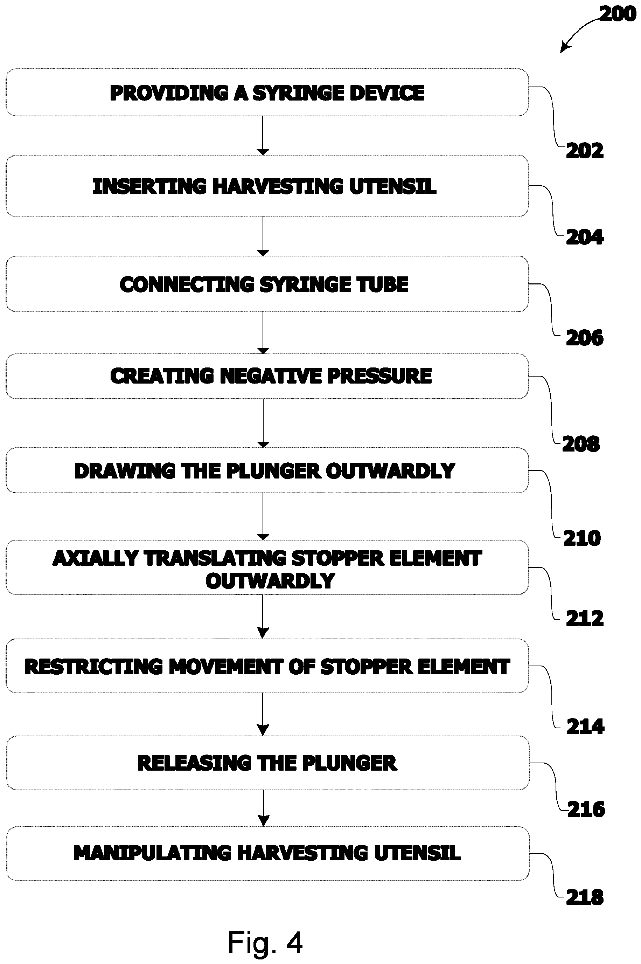

In accordance with some embodiments of the present invention, reference is now made **FIG 4** showing a flowchart of method **200** of harvesting a biological material, such as mammalian fat or any other fatty, fatuous or soft tissue. The method of the embodiment of **FIG 4** illustrates various features that may be interchangeable with elements of any other embodiment described in the specification.

In some embodiments, method **200** commences at step **202** of providing a syringe device. The syringe device comprises a syringe tube, such as syringe tube **12** shown in **FIG 1****.** The syringe tube comprises an essentially elongated cylindrical shell shaped barrel at a proximal portion of the barrel, and at least one finger flange, at a proximal portion of the barrel, configured for manual grip, such as essentially elongated cylindrical shell shaped barrel **14** and at least one finger flange **20** shown in **FIG 1****.**

In some embodiments, the syringe device further includes a plunger, such as plunger **22** shown in **FIG1****.** The plunger comprises an essentially elongated shaft, such as essentially elongated shaft **24** shown in **FIG 1****,** and a piston, such as piston **26** shown in **FIG 1****,** at a distal portion of the elongated shaft, comprising at least one sealing element, configured to form a sealing arrangement with an interior surface of the barrel, whilst contiguously translatable therein.

In some embodiments, the piston of the plunger comprises a fastener part, whereas the distal portion of the elongated shaft comprises a respective fastener part, rendering the piston of the plunger controllably connectable to and disconnectable from the distal portion of the elongated shaft of the plunger, configured for controllably connecting and disconnecting the piston of the plunger to and from the distal portion the said elongated shaft of the plunger.

In some embodiments, the plunger further includes a thumb rest, such as thumb rest **32** shown in **FIG 1****,** at a distal portion of the elongated shaft, configured for exerting a manual pressing force, onto the plunger. In some embodiments, the plunger further comprises at least one recess, such as recess **34** shown in **FIG 1****,** in the essentially elongated shaft of the plunger, where the recess comprises an interior void volume, oriented essentially orthogonally to a longitudinal centerline of the essentially elongated shaft of the plunger.

In some embodiments, the syringe device further includes at least one detent assembly, such as at least one detent assembly **40** shown in **FIG 2B****.** At least one detent assembly comprises a stopper element, such as stopper element **42** shown in **FIG 2B****,** accommodatable within at least one recess in the essentially elongated shaft of the plunger.

In some embodiments, at least one detent assembly further includes a biasing means, such as biasing means **44** shown in **FIG 2B****,** configured for spontaneously driving the stopper element outwardly from at least one recess in the essentially elongated shaft of the plunger. In some embodiments, at least one detent assembly further includes at least one retaining element, such as retaining element **46** shown in **FIG 2B****,** configured for restricting a movement of the stopper element within at least one recess in the essentially elongated shaft of the plunger, thereby preventing the stopper element from leaving at least one recess.

In some embodiments, method **200** proceeds to step **204** of inserting a tip of a harvesting utensil into a target biological tissue, such as the subcutaneous adipose tissue. In some embodiments, method **200** further comprises step **206** of connecting the frusto-conical tip of the syringe tube, to the harvesting utensil.

In some embodiments, method **200** further includes step **208** of creating a negative pressure within the syringe tube, whilst the tip of the harvesting utensil is disposed within the target biological tissue. In some embodiments, method **200** comprises step **210** of drawing the plunger outwardly from the syringe tube. In some embodiments, step **208** of creating a negative pressure within the syringe tube is performed by drawing the plunger outwardly from the syringe tube at step **210.** In some embodiments, step **208** of creating a negative pressure within the syringe tube and/or step **210** of drawing the plunger outwardly from the syringe tube is/are performed subsequently to step **204** of inserting a tip of a harvesting utensil into a target biological tissue and step **206** of connecting the frusto-conical tip of the syringe tube, to the harvesting utensil.

In some embodiments, method **200** further comprises step **212** of axially translating the stopper element by the biasing means, outwardly from at least one recess in the essentially elongated shaft of the plunger from, in the direction essentially orthogonal to the longitudinal centerline of the essentially elongated shaft of the plunger. In some embodiments, method **200** further includes step **214** of restricting by the retaining element the movement of the stopper element outwardly from at least one recess in the essentially elongated shaft of the plunger.

In some embodiments, method **200** further comprises step **216** of releasing the plunger. The releasing of the plunger at step **216** is performed subsequently to the axially translating the stopper element, thereby sustaining the negative pressure within the syringe tube. In some embodiments, method **200** further includes step **218** of manipulating the tip of the harvesting utensil within the target biological tissue, whilst the negative pressure is maintained within the syringe tube, thereby harvesting the biological material.

In some embodiments, if the negative pressure during the harvesting procedure is accidently or inadvertently lost, such as due to disconnection of the tubing or exposure of tip of the harvesting utensil to the ambient environment, method **200** further comprises: firstly detaching the syringe device from the harvesting utensil; secondly exerting manual force onto the stopper element and pushing it in the downward direction; thirdly urging the plunger in the forward direction, so as to remove any air that has entered to the syringe device; fourthly attaching the syringe device back to the harvesting utensil, after the tip of harvesting utensil has been inserted into and/or buried within in the target tissue; fifthly re-performing the aforesaid steps, form step **208** of creating a negative pressure and onwards.

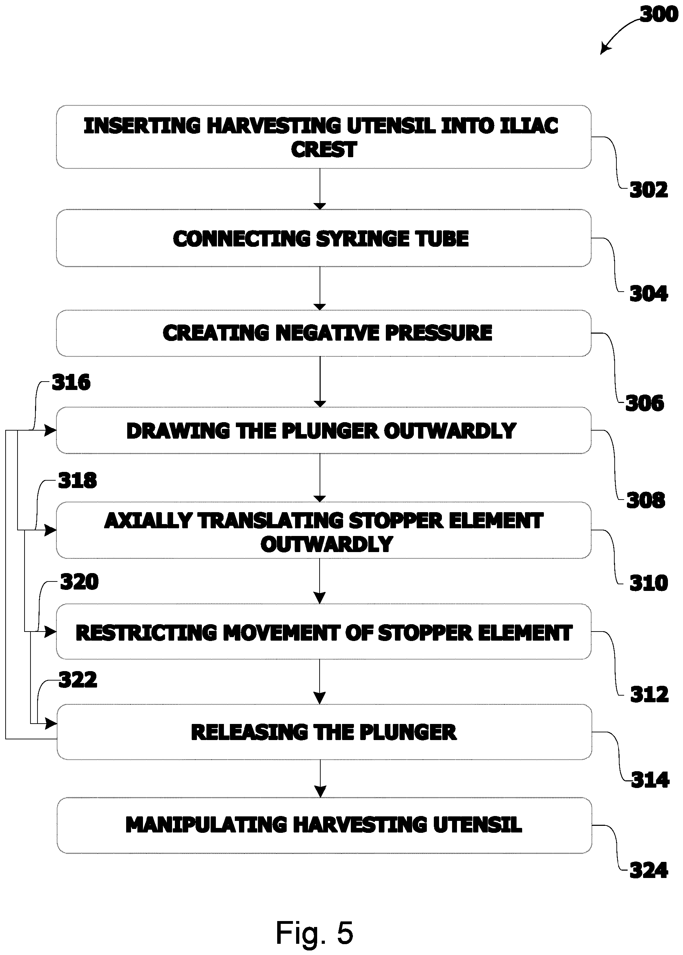

**FIG 5** illustrates another embodiment of the invention. Method **300** of the embodiment of **FIG 5** illustrates various features that may be interchangeable with elements of any other embodiment described in the specification. Method **300** of the embodiment of **FIG 5** provides for an exemplary procedure of bone marrow harvesting with the syringe device shown in **FIG 1** to **2B****.**

In some embodiments, method **300** of bone marrow harvesting with the syringe device comprises step **302** of inserting a tip of a harvesting utensil into the iliac crest. In some examples, the harvesting utensil is an aspiration needle. In some embodiments, once the tip of a harvesting utensil is in the correct position, method **300** further includes step **304** of connecting the frusto-conical tip of the syringe tube to the bone marrow harvesting utensil. In some examples, the syringe tube contains heparin.

In some embodiments, method **300** comprises step **306** of creating a negative pressure within the syringe tube, by drawing a plunger outwardly from the syringe tube, whilst the tip of the bone marrow harvesting utensil is disposed within the iliac crest. In some embodiments, method **300** further proceeds to step **308** of drawing the plunger outwardly from the syringe tube, thereby reaching a first desired volume of the bone marrow.

In some embodiments, method **300** further proceeds to step **310** of axially translating by a biasing means the stopper element of a first detent assembly disposed towards a distal portion of an essentially elongated shaft of the plunger, outwardly from a first recess in the essentially elongated shaft of the plunger from, in a direction essentially orthogonal to a longitudinal centerline of the essentially elongated shaft of the plunger.

In some embodiments, method **300** further proceeds to step **312** of restricting by a retaining element the movement of the first stopper element outwardly from the first recess in the essentially elongated shaft of the plunger. In some embodiments, method **300** further proceeds to step **314** of releasing the plunger, subsequently to the axially translating the first stopper element, thereby sustaining the negative pressure within the syringe tube.

In some embodiments, once the first desired volume of the bone marrow is harvested, method **300** proceeds to step **316** of drawing the plunger outwardly from the syringe tube, thereby reaching a second desired volume of the bone marrow.

In some embodiments, method **300** further proceeds to step **318** of axially translating a second stopper element by a biasing means of a second detent assembly disposed towards a proximal portion of an essentially elongated shaft of the plunger, outwardly from a second recess in the essentially elongated shaft of the plunger from, in a direction essentially orthogonal to a longitudinal centerline of the essentially elongated shaft of the plunger.

In some embodiments, method **300** further proceeds to step **320** of restricting by a retaining element the movement of the second stopper element outwardly from the second recess in the essentially elongated shaft of the plunger. In some embodiments, method **300** further proceeds to step **322** of releasing the plunger, subsequently to the axially translating the second stopper element, thereby sustaining the negative pressure within the syringe tube.

In some embodiments, method **300** further proceeds to step **324** of manipulating the tip of the bone marrow harvesting utensil within the iliac crest, whilst maintaining the negative pressure within the syringe tube, thereby harvesting the bone marrow.

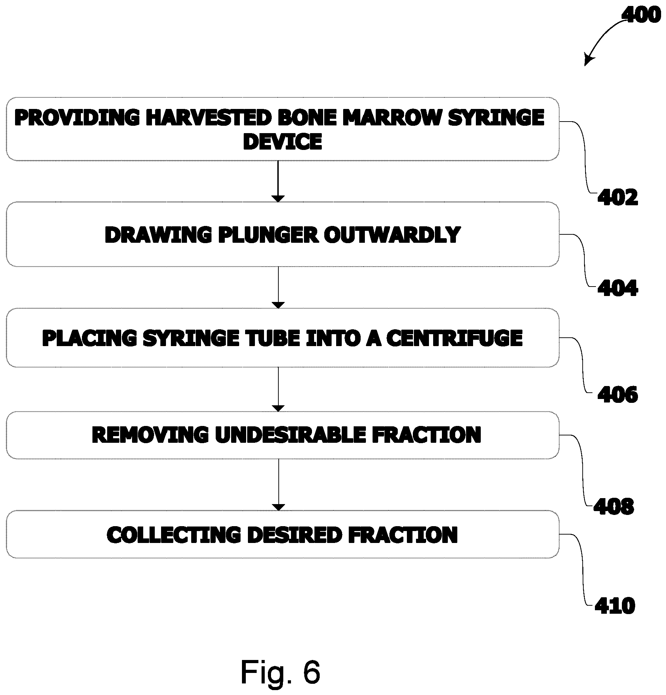

**FIG 6** illustrates yet another embodiment of the invention. Method **400** of the embodiment of **FIG 6** illustrates various features that may be interchangeable with elements of any other embodiment described in the specification. Method **400** of the embodiment of **FIG 6** provides for an exemplary procedure of bone marrow concentration with the syringe device shown in **FIG 1** to **2B****.**

In some embodiment, method **400** commences at step **402** of providing at least one harvested bone marrow syringe device, such as by a means of method **300** shown in **FIG 5****.** In some embodiments, method **400** proceeds to step **404** of drawing a plunger outwardly from a syringe tube of at least one syringe device, while sealing the syringe tube with a sealing element of the plunger.

In some embodiments, method **400** proceeds to step **406** of placing the harvested bone marrow syringe tube within at least one syringe device into a centrifuge and centrifuging it, thereby forming an upper fraction and a lower fraction. In some examples, the harvested bone marrow syringe tube is placed into a centrifuge for a desired number of times and at a desired centrifugal force, forming an upper fraction containing undesired components and a lower fraction containing desired components.

In some embodiments, method **400** proceeds to step **408** of removing from the syringe tube the undesirable fraction, such as a cell free plasma. In some examples, step **408** includes inserting a tip of a harvesting utensil connected to another syringe tube, thereby collecting the cell free plasma.

In some embodiments, method **400** proceeds to step **410** of collecting the desired fraction from the syringe tube. In some examples, step **410** includes inserting a tip of a harvesting utensil through the sealing element of the syringe tube, thereby collecting the buffy coat and leaving the red blood cells formed in the bone marrow in the syringe tube.

In some examples, method **400** comprises a step of transferring the harvested bone marrow contained into the syringe tube, to a separation gel tube, by connecting the syringe device to a tip of a harvesting utensil and inserting it through a separation gel tube rubber stopper. In some examples, method **400** comprises a step pf placing the separation gel tube containing the harvested bone narrow in a centrifuge for separation. In some embodiments, method **400** further proceeds to a step of removing the undesirable components, such as platelet poor plasma, and resuspending the desired components, such as red blood cells formed in the bone marrow, on top of the gel. In some embodiments, method **400** further proceed to a step of collecting the red bloods cells from the fraction below the gel.

### WORKING EXAMPLES

### Example 1 - Exemplary procedure of bone marrow harvesting with the syringe device

In a first working example, a standard bone marrow aspiration needle was inserted into the iliac crest of a patient. Once aspiration needle was in the correct position, the frusto-conical tip of a syringe tube, of standard 20 cc volume, which contained heparin, was connected to the bone marrow aspiration needle.

A negative pressure has then been created within the syringe tube, by drawing the plunger of the syringe device outwardly from the syringe tube, while the tip of the bone marrow aspiration needle remained within the iliac crest. The plunger was removed outwardly from the syringe tube, reaching a first volume of 10 cc of bone marrow.

Subsequently to that, a first stopper element has then been translated by a biasing means of a first detent assembly disposed towards a distal portion of an essentially elongated shaft of the plunger, outwardly from a first recess in the essentially elongated shaft of the plunger, in a direction essentially orthogonal to a longitudinal centerline of the essentially elongated shaft of the plunger.

The movement of the first stopper element outwardly from the first recess in the essentially elongated shaft of the plunger was restricted by a retaining element. Then, subsequently to the axially translating the first stopper element, the plunger was released, thereby sustaining the negative pressure within the syringe tube.

Once the first desired volume of the bone marrow has been harvested the plunger was once again pulled yet further outwardly from the syringe tube, thereby reaching a second volume of 20 cc of bone marrow. A second stopper element then has also been translated by a biasing means of a second detent assembly, disposed towards a distal portion of an essentially elongated shaft of the plunger, outwardly from a second recess in the essentially elongated shaft of the plunger from, in a direction essentially orthogonal to a longitudinal centerline of the essentially elongated shaft of the plunger.

The movement of the second stopper element was also restricted by a retaining element outwardly from the second recess in the essentially elongated shaft of the plunger. Then, subsequently to the axially translating the second stopper element the plunger was released for the second time, thereby sustaining the negative pressure within the syringe tube.

The bone marrow aspiration needle has then been further manipulated within the iliac crest, whilst sustaining the negative pressure within the syringe tube, thereby harvesting the bone marrow, util reaching a second harvested volume of 20 cc.

### Example 2 - Exemplary procedure of bone marrow concentration with the syringe device

A harvested bone marrow, by a means of procedure of **Example 1** , with the syringe device has been provided. Then, the plunger of the syringe device was detached from the syringe tube of the syringe device, by unscrewing the piston from the shaft of the plunger, while maintaining the syringe tube remained closed with a sealing element of the piston.

The harvested bone marrow syringe tube of the syringe device was then placed into a centrifuge for 5 minutes at 800g, forming an upper fraction containing cells free plasma and a lower fraction containing a buffy coat.

The undesirable fraction which contains cells free plasma was withdrawn from the syringe tube, by inserting a 90mm sharp needle connected to an empty regular 20ml syringe, through the sealing element of the piston, thereby collecting the upper fraction, whilst leaving approximately 1ml of plasma above the fraction of the red cells. The 20ml syringe containing the cells free plasma was thereafter detached from the 90mm sharp needle.

The desired fraction was finally collected from the syringe tube, by inserting a sharp needle through the sealing element of a 10ml syringe, thereby collecting the buffy coat.

### INDEX OF REFERENCE NUMERALS

Within the specification hereinabove *inter alia* the following numerals were used to denote the particular constituents in the appended drawings:
**10** - syringe device
**12** - syringe tube
**14** - elongated cylindrical shell shaped barrel
**16** - frusto-conical tip
**18** - distal portion
**19** - finger flange
**20** - proximal portion
**22** - plunger
**24** - elongated shaft
**26** - piston
**28** - distal portion
**29** - fastener moiety
**30** - proximal portion
**32** - thumb rest
**34** - recess
**36** - arrow
**38** - longitudinal centerline
**40** - detent assembly
**42** - stopper element
**43** - structured top surface
**45** - elevation
**44** - biasing means
**46** - retaining element
**100** - process of manufacturing a syringe device
**102** - pre-manufacturing a syringe tube
**104** - molding a plunger
**106** - pre-manufacturing a piston
**108** - mounting the piston onto the distal portion
**110** - molding a stopper element
**112** - pre-manufacturing a biasing means
**114** - assembling at least one detent assembly
**200** - method of harvesting biological material
**202** - providing a syringe device
**204** - inserting a tip of a harvesting utensil
**206** - connecting the frusto-conical tip
**208** - creating a negative pressure
**210** - drawing the plunger outwardly
**212** - axially translating the stopper element
**214** - restricting the stopper element
**216** - releasing the plunger
**218** - manipulating the tip of the harvesting utensil
**300** - bone marrow harvesting
**302** - inserting a tip of a harvesting utensil
**304** - connecting the frusto-conical tip of the syringe
**306** - creating a negative pressure
**308** - drawing the plunger outwardly
**310** - axially translating a first stopper element
**312** - restricting first stopper
**314** - releasing the plunger
**316** - drawing the plunger outwardly
**318** - axially translating a second stopper element
**320** - restricting the movement by retaining element
**322** - releasing the plunger
**324** - manipulating the tip of the bone marrow harvesting utensil
**400** - bone marrow concentration
**402** - providing at least one harvested bone marrow syringe device
**404** - drawing a plunger outwardly
**406** - placing the syringe tube into a centrifuge
**408** - removing undesirable fraction
**410** - collecting the desired fraction

## Claims

1. A syringe device (10) with plunger having axially biased detents (42) comprises:
(a) a syringe tube (12) comprising:
(I) an essentially elongated cylindrical shell shaped barrel (14);
(II) a frusto-conical tip (16), at a distal portion (18) of said barrel (14);
(III) at least one finger flange (19), at a proximal portion (20) of said barrel (14), configured for manual grip;
(b) a plunger (22) comprising:
(I) an essentially elongated shaft (24);
(II) a piston (26) at a distal portion (28) of said elongated shaft (24), comprising at least one sealing element, configured to form a sealing arrangement with an interior surface of said barrel (14), whilst contiguously translatable therein;
(III) a thumb rest (32) at a distal portion (30) of said elongated shaft (24), configured for exerting a manual pressing force, onto said plunger (22);
said syringe device (10) **is characterized by**:
(IV) said plunger (22) comprising at least one recess (34) in said essentially elongated shaft (24) of said plunger (22), wherein said recess (34) comprises an interior void volume, oriented essentially orthogonally (36) to a longitudinal centerline of said essentially elongated shaft (24) of said plunger (22);
(c) at least one detent assembly (40) comprising:
(I) a stopper element (42), accommodatable within said at least one recess (34) in said essentially elongated shaft (24) of said plunger (22);
(II) a biasing means (44), configured for spontaneously driving said stopper element (42) outwardly from said at least one recess (34) in said essentially elongated shaft (24) of said plunger (22);
(III) at least one retaining element (46), configured for restricting a movement of said stopper element (42) within said at least one recess (34) in said essentially elongated shaft (24) of said plunger (22), thereby preventing said stopper element (42) from leaving said at least one recess (34);
wherein said stopper element (42) of said at least one detent assembly (40) is axially translatable within said at least one recess (34), in a direction essentially orthogonal (36) to said longitudinal centerline of said essentially elongated shaft (24) of said plunger (22).

2. The syringe device (10) according to claim 1, wherein said piston (26) of said plunger (22) comprises a fastener part, whereas said distal portion (28) of said elongated shaft (24) comprises a respective fastener part (29), whereby said piston (26) of said plunger (22) is controllably connectable to and disconnectable from said distal portion (28) of said elongated shaft (24) of said plunger (22).

3. The syringe device (10) according to claim 2, wherein said fastener (29) comprises a screw threading.

4. The syringe device (10) according to any of claims 1-3, wherein said at least one detent assembly (40) comprises a first detent assembly (40) and a second detent assembly (40), wherein said first detent assembly (40) is disposed towards said distal portion (28) of said shaft (24), whereas said second detent assembly (40) is disposed towards said proximal portion (30) of said shaft (24).

5. The syringe device (10) according to any of claims 1-4, wherein said stopper element (42), of said at least one detent assembly (40), comprises a structured top surface (43).

6. The syringe device (10) according to claim 5, wherein said structured top surface (43) of said stopper element (42) comprises an elevation (45) at a distal portion thereof.

7. The syringe device (10) according to any of claims 1-6, wherein said retaining element (46) is a pawl, notch, recess, groove or snap.

8. The syringe device (10) according to any of claims 1-6, wherein said retaining element (46) forms an integral part of said essentially elongated shaft (24) of said plunger (22) or forms an integral part of said stopper element (42).

9. The syringe device according to any of claims 1-6, wherein said retaining element (46) further comprises an external retaining element, which forms an integral part neither of said essentially elongated shaft (24) of said plunger (22) nor of said stopper element (42).

10. A method (200) of harvesting biological material comprises the steps of:
(a) providing (202) a syringe device (10) comprising:
(I) a syringe tube (12) comprising:
(i) an essentially elongated cylindrical shell shaped barrel (14);
(ii) a frusto-conical tip (16), at a distal portion (18) of said barrel (14);
(iii) at least one finger flange (19), at a proximal portion (20) of said barrel (14), configured for manual grip;
(II) a plunger (22) comprising:
(i) an essentially elongated shaft (24);
(ii) a piston (26) at a distal portion (28) of said elongated shaft (24), comprising at least one sealing element, configured to form a sealing arrangement with an interior surface of said barrel (14), whilst contiguously translatable therein;
(iii) a thumb rest (32) at a distal portion (30) of said elongated shaft (24), configured for exerting a manual pressing force, onto said plunger (22);
said syringe device (10) **is characterized by**:
(iv) said plunger (22) comprising at least one recess (34) in said essentially elongated shaft (24) of said plunger (22), wherein said recess (34) comprises an interior void volume, oriented essentially orthogonally (36) to a longitudinal centerline of said essentially elongated shaft (24) of said plunger (22);
(III) at least one detent assembly (40) comprising:
(i) a stopper element (42), accommodatable within said at least one recess (34) in said essentially elongated shaft (24) of said plunger (22);
(ii) a biasing means (44), configured for spontaneously driving said stopper element (42) outwardly from said at least one recess (34) in said essentially elongated shaft (24) of said plunger (22);
(iii) at least one retaining element (46), configured for restricting a movement of said stopper element (42) within said at least one recess (34) in said essentially elongated shaft (24) of said plunger (22), thereby preventing said stopper element (42) from leaving said at least one recess (34);
wherein said stopper element (42) of said at least one detent assembly (40) is axially translatable within said at least one recess (34), in a direction essentially orthogonal (36) to said longitudinal centerline of said essentially elongated shaft (24) of said plunger (22);
(b) inserting (204) a tip of a harvesting utensil into a target biological tissue;
(c) connecting (206) said frusto-conical tip (16) of said syringe tube (12), to said harvesting utensil;
(d) creating (208) a negative pressure within said syringe tube (12), by drawing said plunger (22) outwardly from said syringe tube (12), whilst said tip of said harvesting utensil is disposed within said target biological tissue;
(e) drawing (210) said plunger (22) outwardly from said syringe tube (12);
(f) axially translating (212) said stopper element (42) by said biasing means (46), outwardly (36) from said at least one recess (34) in said essentially elongated shaft (24) of said plunger (22), in said direction essentially orthogonal (36) to said longitudinal centerline of said essentially elongated shaft (24) of said plunger (22);
(g) restricting (214) by said retaining element (46) said movement of said stopper element outwardly (36) from said at least one recess (34) in said essentially elongated shaft (24) of said plunger (22);
(h) releasing (216) said plunger (22), subsequently to said axially translating (212) said stopper element (42), thereby sustaining said negative pressure within said syringe tube (12);
(i) manipulating (218) said tip of said harvesting utensil within said target biological tissue, whilst sustaining said negative pressure within said syringe tube (12), thereby harvesting said biological material.

11. The method (200) according to claim 10, wherein said piston (26) of said plunger (22) comprises a fastener part, whereas said distal portion (28) of said elongated shaft (24) comprises a respective fastener part (29), whereby said piston (26) of said plunger (22) is controllably connectable to and disconnectable from said distal portion (28) of said elongated shaft (24) of said plunger (22).

12. The method (200) according to claim 10 or 11, wherein said fastener (29) comprises a screw threading.

13. The method (200) according to any of claims 10-12, wherein said at least one detent assembly (40) comprises a first detent assembly (40) and a second detent assembly (40), wherein said first detent assembly (40) is disposed towards said distal portion (28) of said shaft (24), whereas said second detent assembly (40) is disposed towards said proximal portion (30) of said shaft (24).

14. The method (200) according to any of claims 10-13, wherein said retaining element (46) forms an integral part of said essentially elongated shaft (24) of said plunger (22) or forms an integral part of said stopper element (42).

15. The method (200) according to any of claims 10-13, wherein said retaining element (46) further comprises an external retaining element, which forms an integral part neither of said essentially elongated shaft (24) of said plunger (22) nor of said stopper element (42).
